# EUROPEAN PATENT SPECIFICATION

(11) **EP 3 849 567 B1**
(45) Date of publication and mention of the grant of the patent: **30.10.2024**
(21) Application number: 19860532.1
(22) Date of filing: 11.09.2019
(51) Int. Cl.: C07F 5/02, A61K 33/22, A61P 35/00

(54) **ESTROGEN RECEPTOR TARGETING ANTAGONISTS**
AUF DEN ÖSTROGENREZEPTOR ABZIELENDE ANTAGONISTEN
ANTAGONISTES CIBLANT LE RÉCEPTEUR DES OESTROGÈNES

(30) Priority: 12.09.2018 US 201862730464 P
(43) Date of publication of application: 21.07.2021
(73) Proprietor: Xavier University Of Louisiana, New Orleans, LA 70125 (US)
(72) Inventor: WANG, Guangdi, New Orleans, LA 70122 (US); MOTTAMAL, Madhusoodanan, New Orleans, LA 70125 (US); KANG, Borui, New Orleans, LA 70125 (US)
(74) Representative: Murgitroyd & Company
(86) International application number: PCT/US2019/050573
(87) International publication number: WO 2020/055973

(56) References cited:
- WO-A1-2013/134230
- WO-A1-2013/134230
- WO-A1-2016/196342
- WO-A1-2016/196342
- WO-A1-92/06068
- WO-A2-00/25767
- US-B1- 8 063 249
- DATABASE CAPLUS [online] STN; 1 January 2011 (2011-01-01), KUSHNER P: "Substituted triphenyl butenes, and their preparation and use for the treatment of breast cancer", XP055920393, accession no. US 8063249 B1 Database accession no. 2011:1497183
- PUBCHEM: "(E)-4-[2-[4-[(Z)-1-(4-hydroxyphenyl)-2-phenylbut-1-enyl]phenoxy]ethyl-methylamino]-N-methylbut-2-enamide | C30H34N2O3 - PubChem", WO-2016196342-A1, 18 February 2017 (2017-02-18), pages 1 - 8, XP055920527, Retrieved from the Internet <URL:https://pubchem.ncbi.nlm.nih.gov/compound/124116274> [retrieved on 20220512]

## Description

### BACKGROUND

### 1. Field

The present disclosure relates to compounds that bind competitively to the estrogen receptor and antagonize, down regulate, or covalently bind to the receptor, or act through a combination of these mechanisms of action, thereby blocking the estrogen signaling pathways and inhibit the growth of ER dependent breast cancer cells. The disclosure also relates to pharmaceutical compositions comprising these ER targeting compounds, and such compounds or compositions for use in the treatment of estrogen receptor mediated pathological developments, including cancers.

The references to methods of treatment in this description are to be interpreted as references to the compounds, pharmaceutical compositions and medicaments of the present invention for use in a method for treatment of the human body by therapy,

The present disclosure relates to compounds having activities as selective estrogen receptor modulators (SERMs), as selective estrogen receptor down-regulators (SERDs), and as selective estrogen receptor covalent antagonists, and methods for making the same. The disclosure also relates to pharmaceutical compositions comprising these ER-targeting compounds, and methods for using the same for treatment of estrogen receptor mediated pathological developments, including cancers.

The compounds described here can provide effective endocrine therapy for breast cancers, especially those that express estrogen receptor (estrogen receptor positive or "ER+" breast cancers), as the first line adjuvant treatment regimen, or in the second-line setting as treatment for patients with disease progression after prior endocrine therapy such as selective estrogen receptor modulators (SERMs), aromatase inhibitors (Als), SERDs, or combinations of such endocrine therapies with other anticancer agents.

### 2. Description of Related Art

Breast cancer remains the most common cancer in women worldwide, with over 1.7 million new cases diagnosed in 2012 (second most common cancer overall). This represents about 12% of all new cancer cases and 25% of all cancers in women. Nearly 80% of breast cancer cases are estrogen receptor positive (ER+) [1, 2] and for most of these patients, endocrine therapy is an appropriate option in both the adjuvant and advanced setting. Current endocrine therapy for ER+ breast cancer comprises three regimen options that can be used in varied sequences for optimal outcome: SERM (e.g., tamoxifen, raloxifene, toremifene), aromatase inhibitors (Als, including anastrozole, exemestane, letrozole), and SERD (fulvestrant) [3]. Tamoxifen is a first-line agent for pre-menopausal patients and for women requiring secondary chemoprevention after a DCIS diagnosis. In postmenopausal women Als are generally preferred to tamoxifen because of more favorable time to progression and less severe side effects [4, 5]. However, most patients with advanced metastatic breast cancer eventually develop resistance to tamoxifen or AI treatment while retaining the expression of ERα in the recurrent and/or progressive disease. This clinical information provides a viable therapeutic rationale for using effective ER-targeting antagonists that are not cross-resistant to previous endocrine agents.
WO 2013/134230 discloses boron based 4-hydroxytamoxifen and endoxifen derivatives and their use in treating breast cancer. Kushner P, "Substituted triphenyl butenes, and their preparation and use for the treatment of breast cancer", CAPLUS, STN, (20110101), Database accession no. 2011:1497183 discloses use of a substituted triphenyl butene or prodrug thereof for the treatment of breast cancer in mono-therapy or in combination therapy, or for a reduction in the recurrence rate of previously-treated breast cancer. WO 2016/196342 discloses tetrasubstituted alkene compounds and their use in breast cancer treatment.

### BRIEF SUMMARY

The present disclosure relates generally to novel compounds and compositions useful for the inhibition of estrogen receptor-mediated proliferation (i.e., having activities as selective estrogen receptor modulators (SERMs), as selective estrogen receptor down-regulators (SERDs), and as selective estrogen receptor covalent antagonists); compounds, intermediates, and methods of making such compounds and compositions; methods of using such compounds and compositions; pharmaceutical compositions comprising such compounds and compositions; and methods of using such pharmaceutical compositions, among other things.

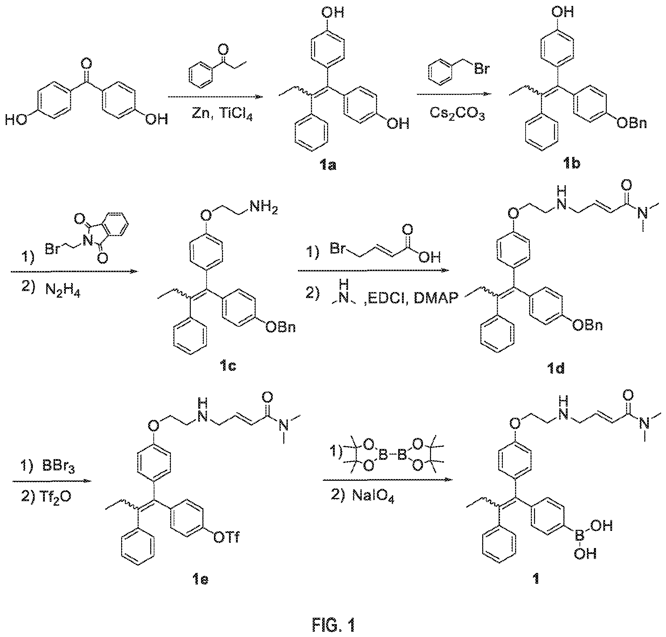

It will be appreciated that the scope is in accordance with the claims. Accordingly, there is provided, as defined in claim 1, a compound of the formula (I): 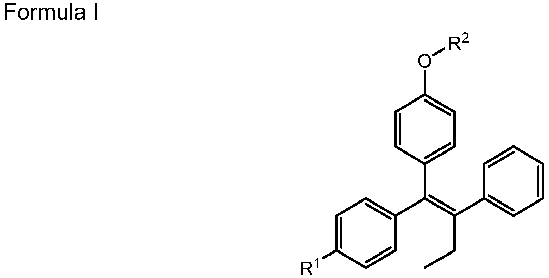 Where:
R¹= 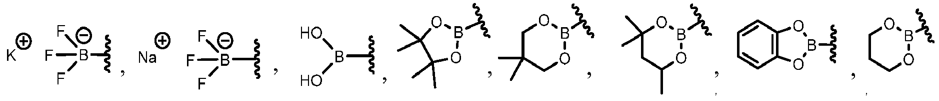
R²= 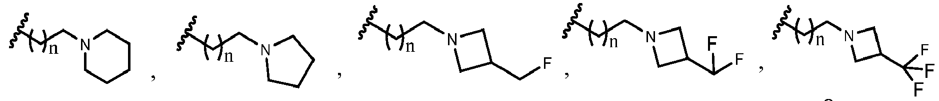 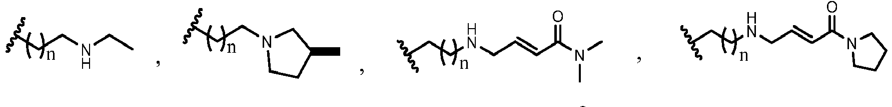 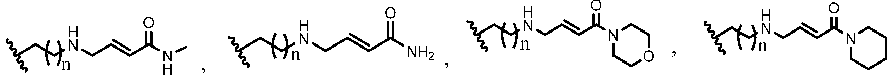 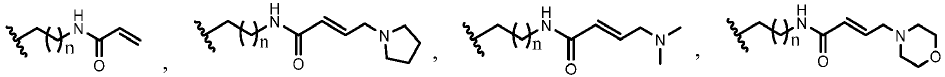
n = 1-3
wherein the R¹ substituent point of attachment is on the substituent boron atom of R¹, or a salt thereof, a solvate thereof, or a solvate of a salt thereof. An example of a compound of Formula (I) is **Compound 1,** and an exemplary scheme for synthesizing **Compound 1** of Formula (I) is shown in FIG. 1. Further features are provided in accordance with the dependent claims, The specification may include description of embodiments outside the scope of the claims provided as background and to assist in the understanding the invention.

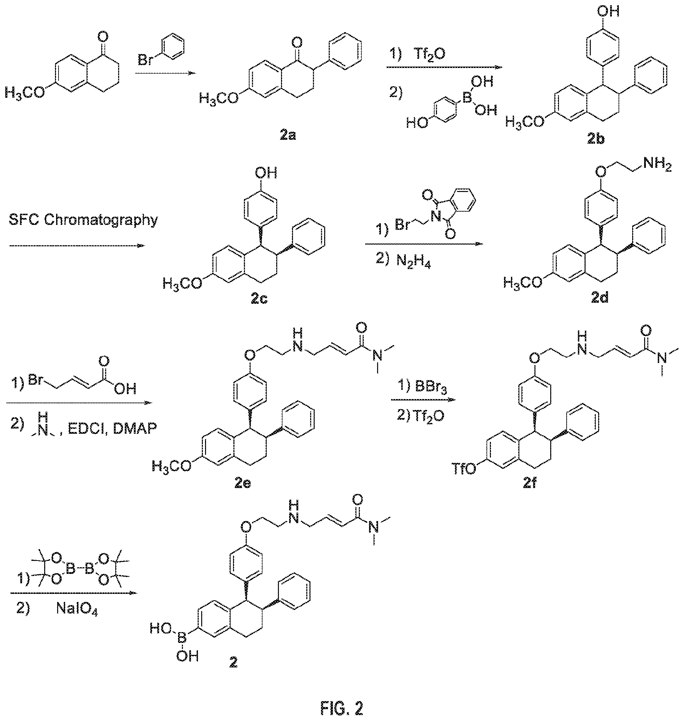

In another embodiment, a compound of the formula (II) is disclosed: 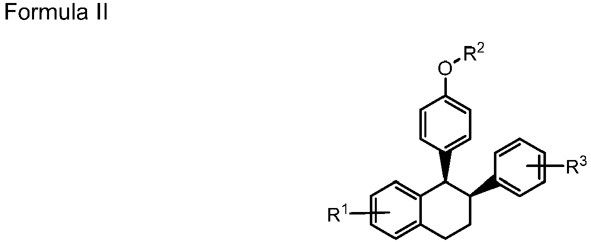
Where R¹ and R³ are para- or meta- substitutions :
   R¹= 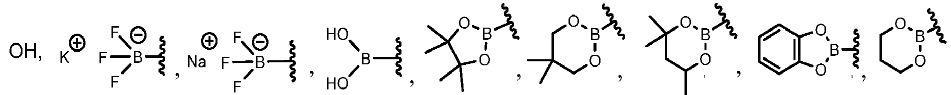
   R²= 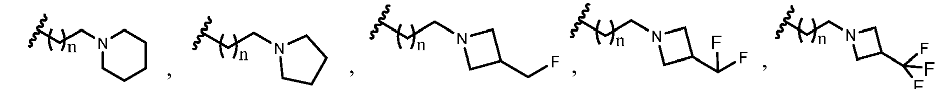 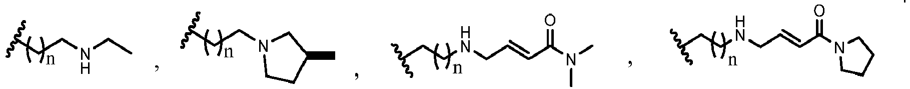 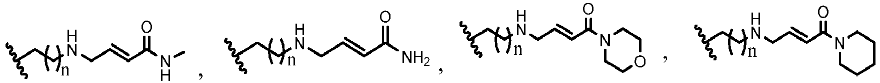 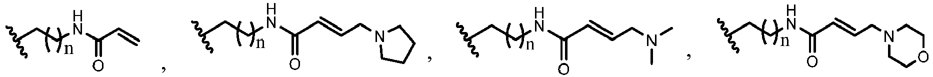 n = 1-3
   R³= H, OH, OCH3, F, or R¹
wherein the R¹ substituent point of attachment is on the substituent boron atom of R¹. An example of a compound of Formula (II) is **Compound 2,** and an exemplary scheme for synthesizing **Compound 2** of Formula (II) is shown in FIG. 2.

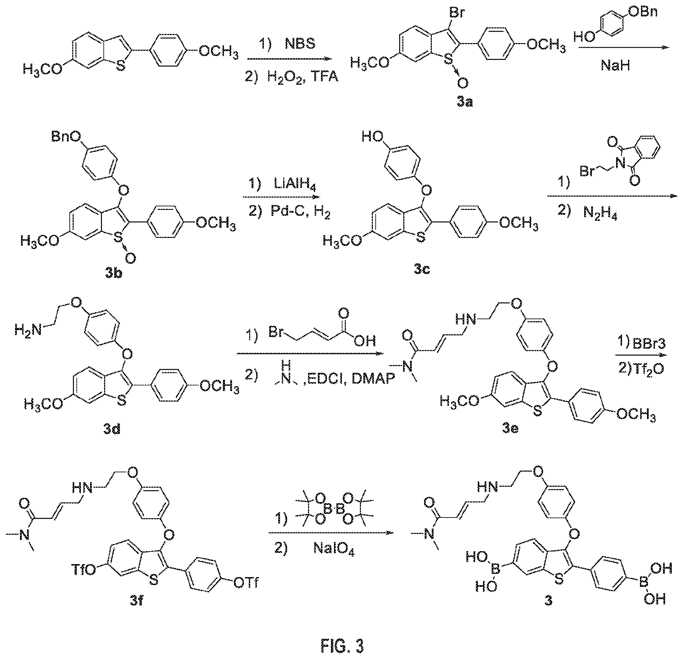

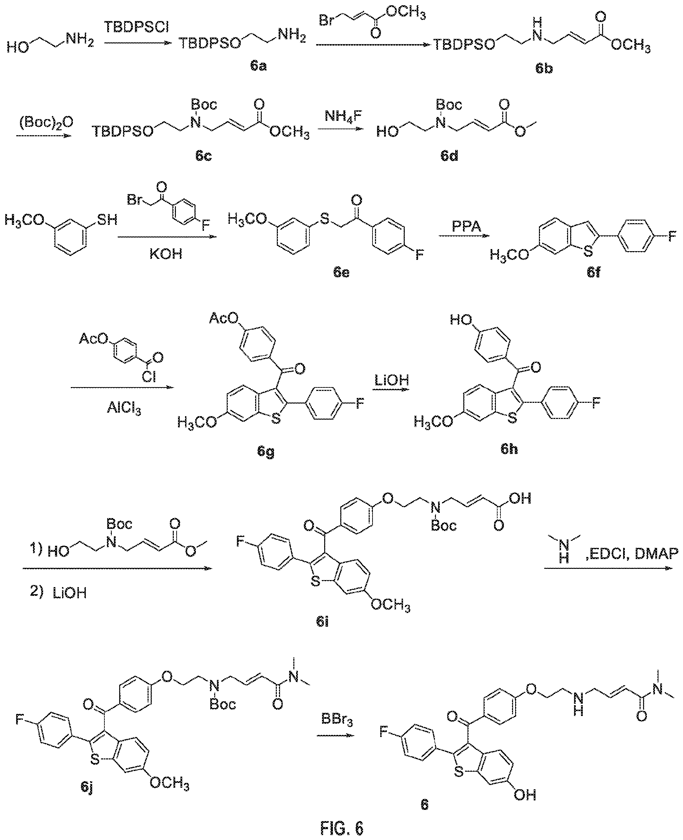

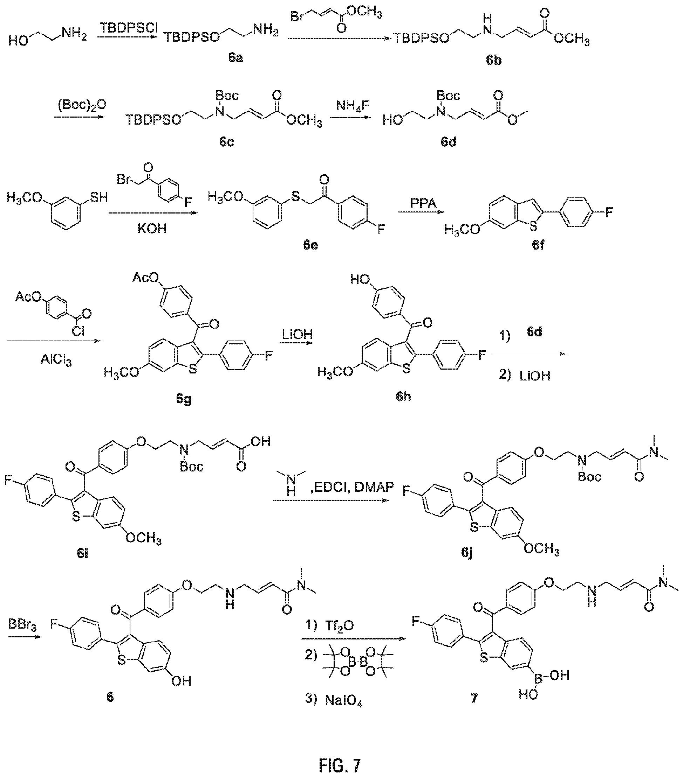

In another embodiment disclosed but not part of the invention, a compound of formula (III) is disclosed: 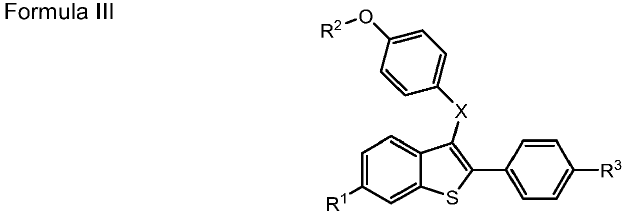 Where:
R¹=OH, 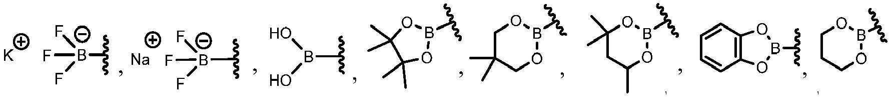
R²= 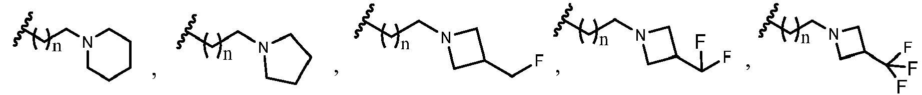 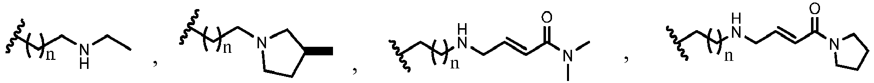 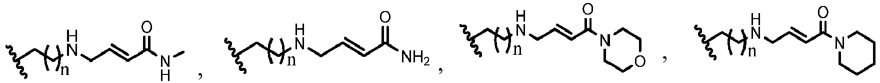 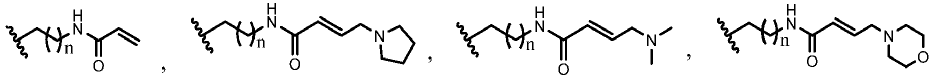 n = 1-3
R³=Cl, F, OH, OCH₃, or R¹
X = O, C=O, NH, CH₂, or absent
wherein the R¹ substituent point of attachment is on the substituent boron atom of R¹. Examples of compounds of Formula (III) are **Compounds 3-8, 15, 16,** and exemplary schemes for synthesizing **Compounds 3-8, 15, 16** of Formula (III) are shown in FIG. 3, FIG. 4, FIG. 5, FIG. 6, FIG. 7, FIG. 8, and FIG. 15, for example.

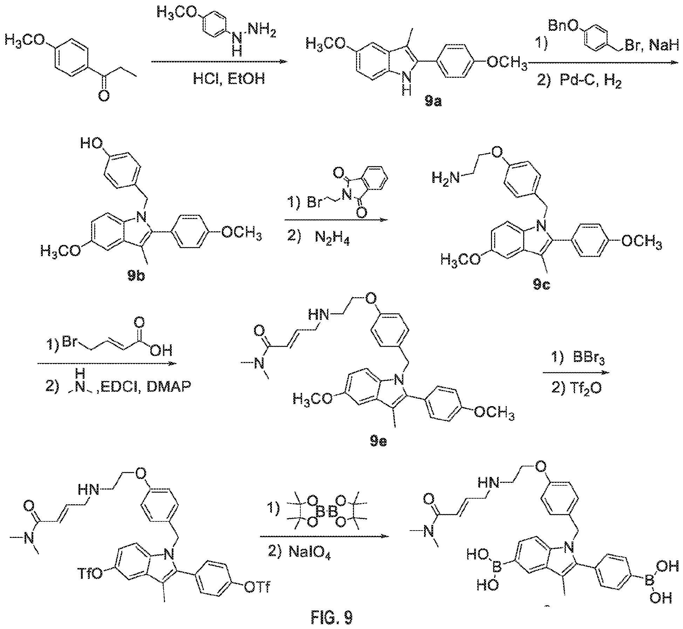

In another embodiment disclosed but not part of the invention, a compound of formula (IV) is disclosed: 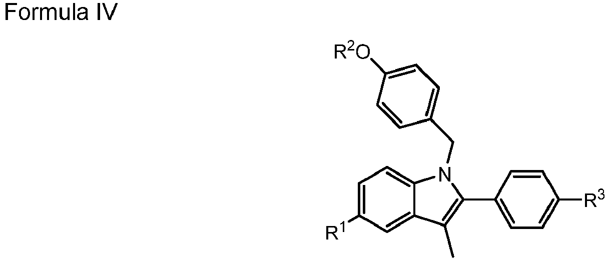 Where:
R¹=OH, 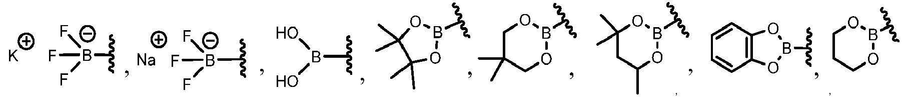
R²= 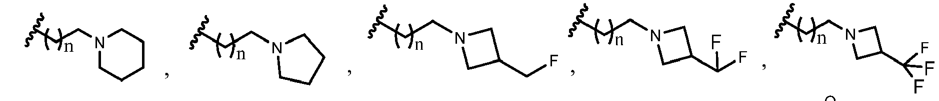 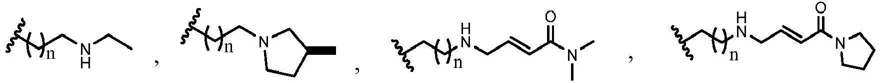 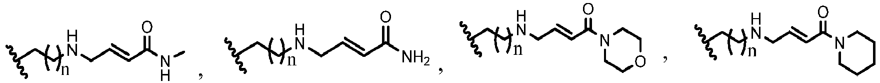 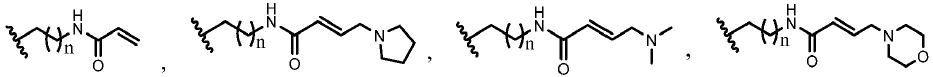 n = 1-3
R³=Cl, F, OH, OCH₃, or R¹
wherein the R¹ substituent point of attachment is on the substituent boron atom of R¹. Examples of compounds of Formula (IV) are **Compounds 9-12,** and exemplary schemes for synthesizing **Compounds 9-12** of Formula (IV) are shown in FIGS. 9, 10, 11, and 12.

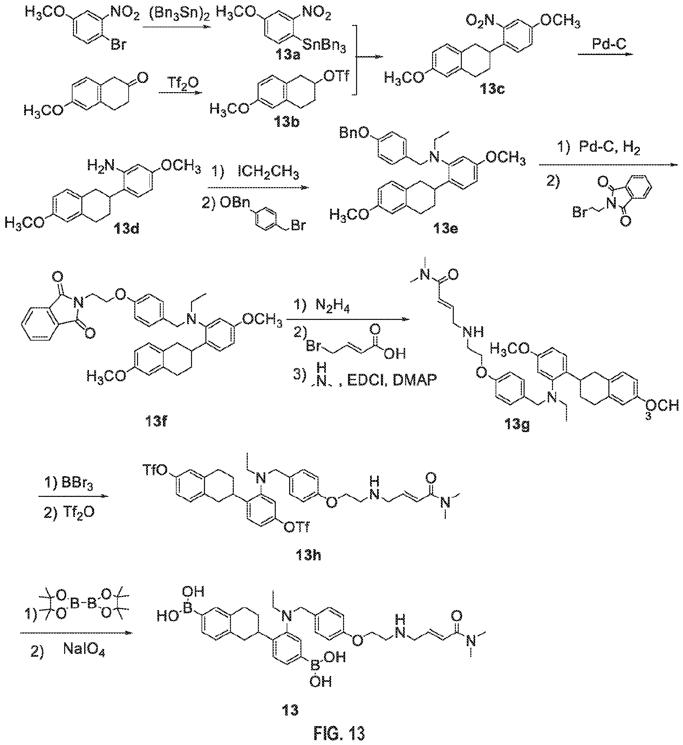

In another embodiment disclosed but not part of the invention, a compound of formula (V) is disclosed: 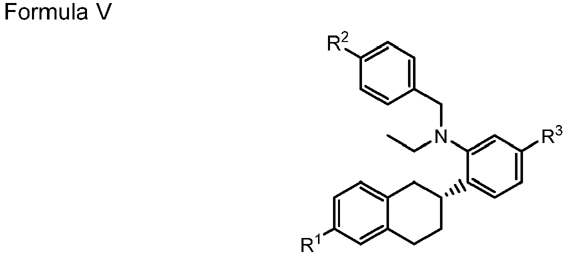 Where:
R¹=OH, 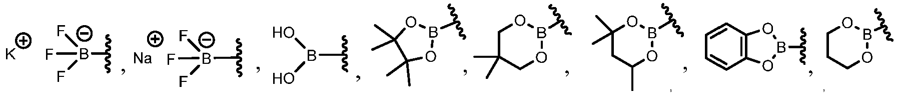
R²= 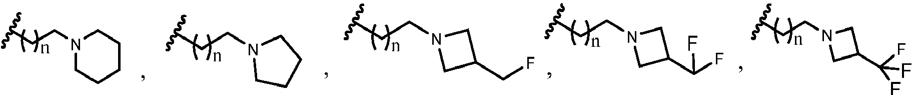 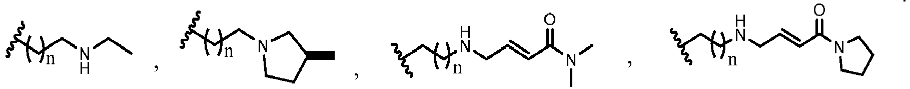 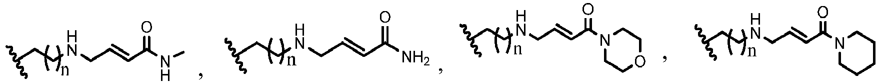 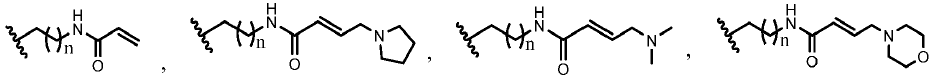 n = 1-3
R³=OH, OCH₃, F, Cl, or R¹
wherein the R¹ substituent point of attachment is on the substituent boron atom of R¹. An example of a compound of Formula (V) is **Compound 13,** and an exemplary scheme for synthesizing Compound 13 of Formula (V) is shown in FIG. 13.

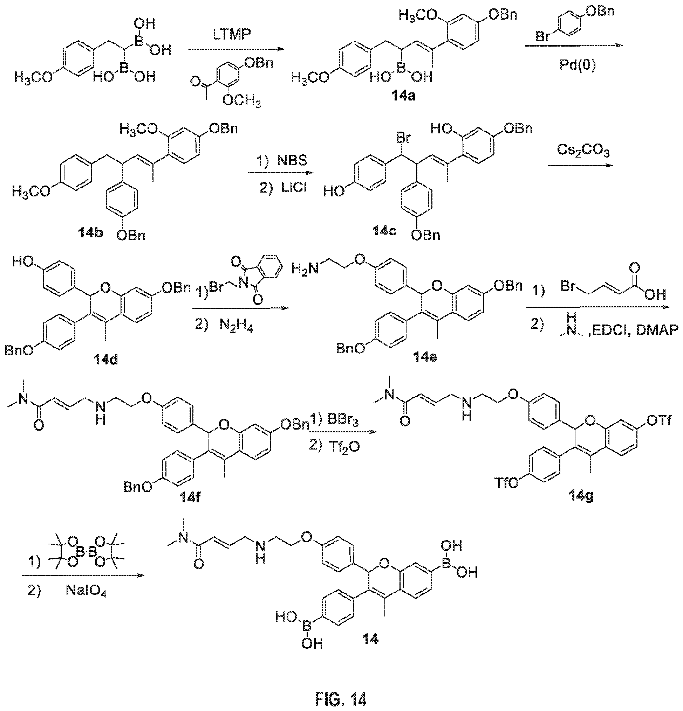

In another embodiment disclosed but not part of the invention, a compound of formula (VI) is disclosed: 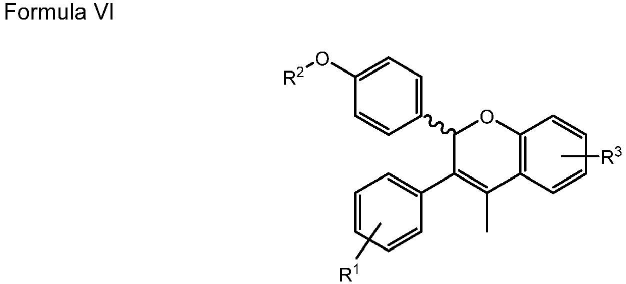 Where R1 and R3 are meta or para substitutions, and:
R¹=OH, 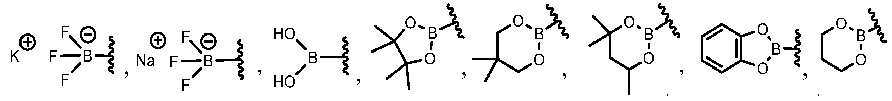
R²= 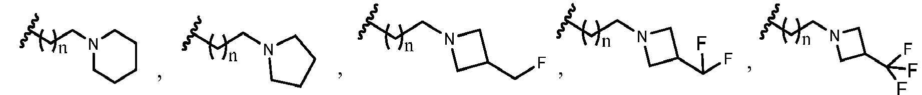 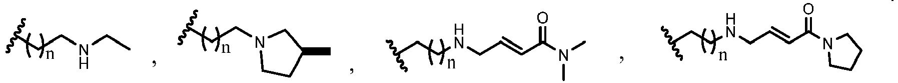 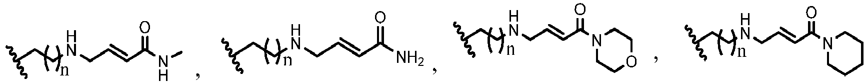 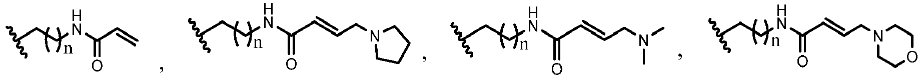 n = 1-3
R³=OH, OCH₃, or R¹
wherein the R¹ substituent point of attachment is on the substituent boron atom of R¹, as depicted more fully by the example compound structures provided below. An example of a compound of Formula (VI) is **Compound 14**, and an exemplary scheme for synthesizing **Compound 14** of Formula (VI) is shown in FIG. 14.

In an embodiment, the disclosure provides for a pharmaceutical composition comprising a compound of Formula (I) as claimed or at least one compound of one of Formulas (I) and (II)-(VI) or a pharmaceutically acceptable salt or solvate thereof. In an embodiment, the pharmaceutical compound is for use in treatment of a proliferative disease, such as a cancer, for example, a breast cancer. A further embodiment may provide a method of treating breast cancer comprising administering to a subject a compound according to any one of the preceding paragraphs. The breast cancer may be an ER-positive breast cancer. The subject may express a mutant ER-α protein. An embodiment may provide use of a compound as in the paragraphs above for treating breast cancer. In some embodiments the breast cancer is an ER-positive breast cancer. In some embodiments said subject expresses a mutant ER-α protein. In some embodiments a compound as presented above is used in the preparation of a medicament for treatment of breast cancer.

The pharmaceutical compositions of the present disclosure can be in any form known to those of skill in the art. For instance, in some embodiments the pharmaceutical compositions are in a form of a product for oral delivery, said product form being selected from a group consisting of a concentrate, dried powder, liquid, capsule, pellet, and pill. In other embodiments, the pharmaceutical compositions of the disclosure are in the form of a product for parenteral administration including intravenous, intradermal, intramuscular, and subcutaneous administration. The pharmaceutical compositions disclosed herein may also further comprise carriers, binders, diluents, and excipients.

Also, in other aspects, the present disclosure relates to new ER-targeting composition comprising a compound of Formula (I) as claimed or one or more compounds selected from the group consisting of a compound of Formula (I) and one of Formula (II) through (VI), and a compound of one of Compound 1 as claimed through Compound 16, and pharmaceutically acceptable salts and solvates thereof. In an embodiment, said compound has a purity of ≥ 75%, ≥ 80%, ≥ 85%, ≥ 90%, ≥ 95%, ≥ 96%, ≥ 97%, or ≥ 98%, and ≥ 99%. In an embodiment, a pharmaceutical composition is provided comprising the new ER-targeting composition, either alone or in combination with at least one additional therapeutic agent, with a pharmaceutically acceptable carrier; and uses of the new ER-targeting compositions, either alone or in combination with at least one additional therapeutic agent, in the treatment of proliferative diseases including breast cancer at any stage of the disease diagnosis. The combination with an additional therapeutic agent may take the form of combining the new ER-targeting compounds with any known therapeutic agent.

The methods for treating a clinical indication by the ER-targeting compounds disclosed herein, may be effectuated by administering a therapeutically effective amount of the ER-targeting compounds to a patient in need thereof, this therapeutically effective amount may comprise administration of the prodrug to the patient at 1 mg/kg/day, 2 mg/kg/day, 3 mg/kg/day, 4 mg/kg/day, 5 mg/kg/day, 10 mg/kg/day and 20 mg/kg/day. Alternatively, amounts ranging from about 0.001 mg/kg/day to about 0.01 mg/kg/day, or about 0.01 mg/kg/day to about 0.1 mg/kg/day, or about 0.1 mg/kg/day to about 1 mg/kg/day, or about 1 mg/kg/day to 10 mg/kg/day, or about 10 mg/kg/day to about 100 mg/kg/day are also contemplated.

A further object of the disclosure is a kit, comprising a composition containing at least one ER-targeting for treatment and prevention of cancer and cancer related morbidities. The composition of the kit may comprise at least one carrier, at least one binder, at least one diluent, at least one excipient, at least one other therapeutic agent, or mixtures thereof.

One aspect of the present disclosure is the compounds disclosed herein as well as the intermediates as used for their synthesis.

These and other features, aspects, and advantages of embodiments of the present disclosure will become better understood with regard to the following descriptions, claims, and accompanying drawings explained below.

### BRIEF DESCRIPTION OF THE DRAWINGS

For a further understanding of the nature, objects, and advantages of the present disclosure, reference should be had to the following detailed description, read in conjunction with the following drawings, wherein like reference numerals denote like elements.
FIG. 1 shows an exemplary scheme for preparation of Compound 1.
FIG. 2 shows an exemplary scheme for preparation of Compound 2.
FIG. 3 shows an exemplary scheme for preparation of Compound 3.
FIG. 4 shows an exemplary scheme for preparation of Compound 4.
FIG. 5 shows an exemplary scheme for preparation of Compound 5.
FIG. 6 shows an exemplary scheme for preparation of Compound 6.
FIG. 7 shows an exemplary scheme for preparation of Compound 7.
FIG. 8 shows an exemplary scheme for preparation of Compound 8.
FIG. 9 shows an exemplary scheme for preparation of Compound 9.
FIG. 10 shows an exemplary scheme for preparation of Compound 10.
FIG. 11 shows an exemplary scheme for preparation of Compound 11.
FIG. 12 shows an exemplary scheme for preparation of Compound 12.
FIG. 13 shows an exemplary scheme for preparation of Compound 13.
FIG. 14 shows an exemplary scheme for preparation of Compound 14.
FIG.15 shows an exemplary scheme for preparation of Compounds 15 and 16.
FIG. 16 shows competitive binding curves of compound 1 and compound 4 to the estrogen receptor.
FIG. 17 shows (A) dose-dependent inhibition of E2 stimulated transcriptional activity by Compound 1; and (B) that pretreatment with Compound 1 allows irreversible binding to ER, and subsequent addition of E2 does not restore ER transcription activity.
FIG. 18 shows dose dependent antiproliferative effect of compound 1 on MCF-7, MCF-7/TamR, and MCF-7/Y537S breast cancer cells.
FIG. 19 shows that compound 1 is effective in inhibiting (A) MCF-7 xenograft tumor growth and (B) growth of ST941/HI PDX breast tumor harboring ESR1 mutant (Y537S).
FIG. 20 shows the uterotrophic effect of compound 1 and compound 4 in mice.
FIG. 21 shows the plasma concentration of compound 1 over time after a single oral dose of 5 mg/kg in Sprague Dawley rats.

### DETAILED DESCRIPTION

Before the subject disclosure is further described, it is to be understood that the disclosure is not limited to the particular embodiments of the disclosure described below, as variations of the particular embodiments may be made and still fall within the scope of the appended claims. It is also to be understood that the terminology employed is for the purpose of describing particular embodiments, and is not intended to be limiting. Instead, the scope of the present disclosure will be established by the appended claims.

In this specification and the appended claims, the singular forms "a," "an," and "the" include plural reference unless the context clearly dictates otherwise. Unless defined otherwise, all technical and scientific terms used herein have the same meaning as commonly understood to one of ordinary skill in the art to which this disclosure belongs.

As used herein, the term "minimize" or "reduce", or derivatives thereof, include a complete or partial inhibition of a specified biological effect (which is apparent from the context in which the terms "minimize" or "reduce" are used).

The compounds according to the disclosure are isolated and purified in a manner known per se, e.g. by distilling off the solvent in vacuo and recrystallizing the residue obtained from a suitable solvent or subjecting it to one of the customary purification methods, such as chromatography on a suitable support material. Furthermore, reverse phase preparative HPLC of compounds of the present disclosure which possess a sufficiently basic or acidic functionality, may result in the formation of a salt, such as, in the case of a compound of the present disclosure which is sufficiently basic, a trifluoroacetate or formate salt for example, or, in the case of a compound of the present disclosure which is sufficiently acidic, an ammonium salt for example. Salts of this type can either be transformed into its free base or free acid form, respectively, by various methods known to the person skilled in the art, or be used as salts in subsequent biological assays. Additionally, the drying process during the isolation of compounds of the present disclosure may not fully remove traces of cosolvents, especially such as formic acid or trifluoroacetic acid, to give solvates or inclusion complexes. The person skilled in the art will recognize which solvates or inclusion complexes are acceptable to be used in subsequent biological assays. It is to be understood that the specific form (e.g., salt, free base, solvate, inclusion complex) of a compound of the present disclosure as isolated as described herein is not necessarily the only form in which said compound can be applied to a biological assay in order to quantify the specific biological activity.

One aspect of the disclosure is salts of the compounds according to the disclosure including all inorganic and organic salts, especially all pharmaceutically acceptable inorganic and organic salts, particularly all pharmaceutically acceptable inorganic and organic salts customarily used in pharmacy.

Examples of salts include, but are not limited to, lithium, sodium, potassium, calcium, aluminum, magnesium, titanium, meglumine, ammonium, salts optionally derived from NH₃ or organic amines having from 1 to 16 C-atoms such as, *e.g.*, ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylendiamine, N-methylpiperindine and guanidinium salts.

The salts include water-insoluble and, particularly, water-soluble salts.

As used herein, "pharmaceutically acceptable salts" refer to derivatives of the compounds disclosed herein wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicyclic, stearic, subacetic, succinic, sulfamic, sulfanilic, sulfuric, tannic, tartaric, toluene sulfonic, and the commonly occurring amine acids, e.g., glycine, alanine, phenylalanine, arginine, etc.

Other examples of pharmaceutically acceptable salts include hexanoic acid, cyclopentane propionic acid, pyruvic acid, malonic acid, 3-(4-hydroxybenzoyl)benzoic acid, cinnamic acid, 4-chlorobenzenesulfonic acid, 2-naphthalenesulfonic acid, 4-toluenesulfonic acid, camphorsulfonic acid, 4-methylbicyclo-[2.2.2]-oct-2-ene-1-carboxylic acid, 3-phenylpropionic acid, trimethylacetic acid, tertiary butylacetic acid, muconic acid, and the like. The present disclosure also encompasses salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, N-methylglucamine, and the like. In the salt form, it is understood that the ratio of the compound to the cation or anion of the salt may be 1:1, or any ratio other than 1:1, e.g., 3:1, 2:1, 1:2, or 1:3.

It should be understood that all references to pharmaceutically acceptable salts include solvent addition forms (solvates) or crystal forms (polymorphs) as defined herein, of the same salt.

Salts of the compounds of formulas (I) as claimed and (II) through (VI) according to the disclosure can be obtained by dissolving the free compound in a suitable solvent (for example a ketone such as acetone, methylethylketone or methylisobutylketone, an ether such as diethyl ether, tetrahydrofuran or dioxane, a chlorinated hydrocarbon such as methylene chloride or chloroform, or a low molecular weight aliphatic alcohol such as methanol, ethanol or isopropanol) which contains the desired acid or base, or to which the desired acid or base is then added. The acid or base can be employed in salt preparation, depending on whether a mono- or polybasic acid or base is concerned and depending on which salt is desired, in an equimolar quantitative ratio or one differing therefrom. The salts are obtained by filtering, reprecipitating, precipitating with a non-solvent for the salt or by evaporating the solvent. Salts obtained can be converted into the free compounds which, in turn, can be converted into salts. In this manner, pharmaceutically unacceptable salts, which can be obtained, for example, as process products in the manufacturing on an industrial scale, can be converted into pharmaceutically acceptable salts by processes known to the person skilled in the art.

According to the person skilled in the art the compounds of formulas (I) as claimed and (II) through (VI) according to this disclosure as well as their salts may contain, *e.g*., when isolated in crystalline form, varying amounts of solvents. Included within the scope of the disclosure are therefore all solvates and in particular all hydrates of the compounds of formulas (I) as claimed and (II) through (VI) according to this disclosure as well as all solvates and in particular all hydrates of the salts of the compounds of formulas (I) as claimed and (II) through (VI) according to this disclosure.

"Solvate" means solvent addition forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate; and if the solvent is alcohol, the solvate formed is an alcoholate. Hydrates are formed by the combination of one or more molecules of water with one molecule of the substance in which the water retains its molecular state as H₂O.

The compounds according to the disclosure and their salts can exist in the form of tautomers which are included in the embodiments of the disclosure.

"Tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solutions where tautomerization is possible, a chemical equilibrium of the tautomers will be reached. The exact ratio of the tautomers depends on several factors, including temperature, solvent and pH. The concept of tautomers that are interconvertible by tautomerizations is called tautomerism.

Where the present specification depicts a compound prone to tautomerization, but only depicts one of the tautomers, it is understood that all tautomers are included as part of the meaning of the chemical depicted. It is to be understood that the compounds disclosed herein may be depicted as different tautomers. It should also be understood that when compounds have tautomeric forms, all tautomeric forms are intended to be included, and the naming of the compounds does not exclude any tautomer form.

Of the various types of tautomerism that are possible, two are commonly observed. In keto-enol tautomerism a simultaneous shift of electrons and a hydrogen atom occurs. Ring-chain tautomerism arises as a result of the aldehyde group (-CHO) in a sugar chain molecule reacting with one of the hydroxy groups (-OH) in the same molecule to give it a cyclic (ring-shaped) form as exhibited by glucose.

Common tautomeric pairs are: ketone-enol, amide-nitrile, lactam-lactim, amide-imidic acid tautomerism in heterocyclic rings (e.g., in nucleobases such as guanine, thymine and cytosine), imine-enamine and enamine-enamine.

The compounds of the disclosure may, depending on their structure, exist in different stereoisomeric forms. These forms include configurational isomers or optically conformational isomers (enantiomers and/or diastereoisomers including those of atropisomers). The present disclosure therefore includes enantiomers, diastereoisomers as well as mixtures thereof. From those mixtures of enantiomers and/or disastereoisomers pure stereoisomeric forms can be isolated with methods known in the art, preferably methods of chromatography, especially high performance liquid chromatography (HPLC) using achiral or chiral phase. The disclosure further includes all mixtures of the stereoisomers mentioned above independent of the ratio, including the racemates.

The compounds of the disclosure may, depending on their structure, exist in various stable isotopic forms. These forms include those in which one or more hydrogen atoms have been replaced with deuterium atoms, those in which one or more nitrogen atoms have been replaced with ¹⁵N atoms, or those in which one or more atoms of carbon, fluorine, chlorine, bromine, sulfur, or oxygen have been replaced by the stable isotope of the respective, original atoms.

Some of the compounds and salts according to the disclosure may exist in different crystalline forms (polymorphs) which are within the scope of the disclosure.

It is a further object of the disclosure to provide ER-targeting compounds, methods of synthesizing the ER-targeting compounds, methods of manufacturing the ER-targeting compounds, and methods of using the ER-targeting compounds.

Another object of the disclosure is to provide a composition, for example a pharmaceutical composition, comprising at least one ER-targeting compound in an amount effective for use in the treatment of proliferative diseases such as cancer, including but not limited to endocrine related cancer. In an embodiment, the cancer is an ER-positive tumor, such as a tumor of the breast, endometrium, uterus, or ovary. In an embodiment, the tumor is an ER-positive tumor of the breast. In an embodiment, the breast tumor is determined to be ER-positive by an immunohistochemical method described by Hammond *et al.* [6].

In an embodiment, the object of such treatment is to inhibit estrogen-induced proliferation of a cell. In a further embodiment, said object is to inhibit estrogen-induced proliferation of a cell by a mechanism selected from SERM, SERD, and SERCA.

As used herein, use in "treating" or 'treatment' means us of a pharmaceutical composition to ameliorate, reduce or lessen the symptoms of a disease. As used herein, "treating" or "treat" describes the management and care of a subject for the purpose of combating a disease, condition, or disorder and includes the use of a compound disclosed herein, or a pharmaceutically acceptable salt, polymorph or solvate thereof, to alleviate the symptoms or complications of a disease, condition or disorder, or to eliminate the disease, condition or disorder. The term "treat" may also include treatment of a cell in vitro or an animal model. As used herein, "subject" or "subjects" refers to any animal, such as mammals including rodents (e.g., mice or rats), dogs, primates, lemurs or humans.

Treating cancer may result in a reduction in size of a tumor. A reduction in size of a tumor may also be referred to as "tumor regression." Preferably, after treatment, tumor size is reduced by 5% or greater relative to its size prior to treatment; more preferably, tumor size is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Size of a tumor may be measured by any reproducible means of measurement. The size of a tumor may be measured as a diameter of the tumor.

Treating cancer may result in a reduction in tumor volume. Preferably, after treatment, tumor volume is reduced by 5% or greater relative to its size prior to treatment; more preferably, tumor volume is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Tumor volume may be measured by any reproducible means of measurement.

Treating cancer may result in a decrease in number of tumors. Preferably, after treatment, tumor number is reduced by 5% or greater relative to number prior to treatment; more preferably, tumor number is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. Number of tumors may be measured by any reproducible means of measurement. The number of tumors may be measured by counting tumors visible to the naked eye or at a specified magnification. Preferably, the specified magnification is 2×, 3×, 4×, 5×, 10×, or 50×.

Treating cancer may result in a decrease in number of metastatic lesions in other tissues or organs distant from the primary tumor site. Preferably, after treatment, the number of metastatic lesions is reduced by 5% or greater relative to number prior to treatment; more preferably, the number of metastatic lesions is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. The number of metastatic lesions may be measured by any reproducible means of measurement. The number of metastatic lesions may be measured by counting metastatic lesions visible to the naked eye or at a specified magnification. Preferably, the specified magnification is 2×, 3×, 4×, 5×, 10×, or 50×.

Treating cancer may result in an increase in average survival time of a population of treated subjects in comparison to a population receiving carrier alone. Preferably, the average survival time is increased by more than 30 days; more preferably, by more than 60 days; more preferably, by more than 90 days; and most preferably, by more than 120 days. An increase in average survival time of a population may be measured by any reproducible means. An increase in average survival time of a population may be measured, for example, by calculating for a population the average length of survival following initiation of treatment with an active compound. An increase in average survival time of a population may also be measured, for example, by calculating for a population the average length of survival following completion of a first round of treatment with an active compound.

Treating cancer may result in an increase in average survival time of a population of treated subjects in comparison to a population of untreated subjects. Preferably, the average survival time is increased by more than 30 days; more preferably, by more than 60 days; more preferably, by more than 90 days; and most preferably, by more than 120 days. An increase in average survival time of a population may be measured by any reproducible means. An increase in average survival time of a population may be measured, for example, by calculating for a population the average length of survival following initiation of treatment with an active compound. An increase in average survival time of a population may also be measured, for example, by calculating for a population the average length of survival following completion of a first round of treatment with an active compound.

Treating cancer may result in increase in average survival time of a population of treated subjects in comparison to a population receiving monotherapy with a drug that is not a compound disclosed herein, or a pharmaceutically acceptable salt thereof. Preferably, the average survival time is increased by more than 30 days; more preferably, by more than 60 days; more preferably, by more than 90 days; and most preferably, by more than 120 days. An increase in average survival time of a population may be measured by any reproducible means. An increase in average survival time of a population may be measured, for example, by calculating for a population the average length of survival following initiation of treatment with an active compound. An increase in average survival time of a population may also be measured, for example, by calculating for a population the average length of survival following completion of a first round of treatment with an active compound.

Treating cancer may result in a decrease in the mortality rate of a population of treated subjects in comparison to a population receiving carrier alone. Treating cancer may result in a decrease in the mortality rate of a population of treated subjects in comparison to an untreated population. Treating cancer may result in a decrease in the mortality rate of a population of treated subjects in comparison to a population receiving monotherapy with a drug that is not a compound disclosed herein, or a pharmaceutically acceptable salt, prodrug, metabolite, analog or derivative thereof. Preferably, the mortality rate is decreased by more than 2%; more preferably, by more than 5%; more preferably, by more than 10%; and most preferably, by more than 25%. A decrease in the mortality rate of a population of treated subjects may be measured by any reproducible means. A decrease in the mortality rate of a population may be measured, for example, by calculating for a population the average number of disease-related deaths per unit time following initiation of treatment with an active compound. A decrease in the mortality rate of a population may also be measured, for example, by calculating for a population the average number of disease-related deaths per unit time following completion of a first round of treatment with an active compound.

Treating cancer may result in a decrease in tumor growth rate. Preferably, after treatment, tumor growth rate is reduced by at least 5% relative to number prior to treatment; more preferably, tumor growth rate is reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. Tumor growth rate may be measured by any reproducible means of measurement. Tumor growth rate may be measured according to a change in tumor diameter per unit time.

Treating cancer may result in a decrease in tumor regrowth, for example, following attempts to remove it surgically. Preferably, after treatment, tumor regrowth is less than 5%; more preferably, tumor regrowth is less than 10%; more preferably, less than 20%; more preferably, less than 30%; more preferably, less than 40%; more preferably, less than 50%; even more preferably, less than 50%; and most preferably, less than 75%. Tumor regrowth may be measured by any reproducible means of measurement. Tumor regrowth is measured, for example, by measuring an increase in the diameter of a tumor after a prior tumor shrinkage that followed treatment. A decrease in tumor regrowth is indicated by failure of tumors to reoccur after treatment has stopped.

Treating or preventing a cell proliferative disorder may result in a reduction in the rate of cellular proliferation. Preferably, after treatment, the rate of cellular proliferation is reduced by at least 5%; more preferably, by at least 10%; more preferably, by at least 20%; more preferably, by at least 30%; more preferably, by at least 40%; more preferably, by at least 50%; even more preferably, by at least 50%; and most preferably, by at least 75%. The rate of cellular proliferation may be measured by any reproducible means of measurement. The rate of cellular proliferation is measured, for example, by measuring the number of dividing cells in a tissue sample per unit time.

Treating or preventing a cell proliferative disorder may result in a reduction in the proportion of proliferating cells. Preferably, after treatment, the proportion of proliferating cells is reduced by at least 5%; more preferably, by at least 10%; more preferably, by at least 20%; more preferably, by at least 30%; more preferably, by at least 40%; more preferably, by at least 50%; even more preferably, by at least 50%; and most preferably, by at least 75%. The proportion of proliferating cells may be measured by any reproducible means of measurement. Preferably, the proportion of proliferating cells is measured, for example, by quantifying the number of dividing cells relative to the number of nondividing cells in a tissue sample. The proportion of proliferating cells may be equivalent to the mitotic index.

Treating or preventing a cell proliferative disorder may result in a decrease in size of an area or zone of cellular proliferation. Preferably, after treatment, size of an area or zone of cellular proliferation is reduced by at least 5% relative to its size prior to treatment; more preferably, reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. Size of an area or zone of cellular proliferation may be measured by any reproducible means of measurement. The size of an area or zone of cellular proliferation may be measured as a diameter or width of an area or zone of cellular proliferation.

Treating or preventing a cell proliferative disorder may result in a decrease in the number or proportion of cells having an abnormal appearance or morphology. Preferably, after treatment, the number of cells having an abnormal morphology is reduced by at least 5% relative to its size prior to treatment; more preferably, reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. An abnormal cellular appearance or morphology may be measured by any reproducible means of measurement. An abnormal cellular morphology may be measured by microscopy, e.g., using an inverted tissue culture microscope. An abnormal cellular morphology may take the form of nuclear pleiomorphism.

### EXAMPLES

Hereby are provided non-limiting examples of embodiments of compounds disclosed herein.

### Example 1

As an illustrative example of a compound of formula (I), the following compound, denoted **Compound 1,** was synthesized:

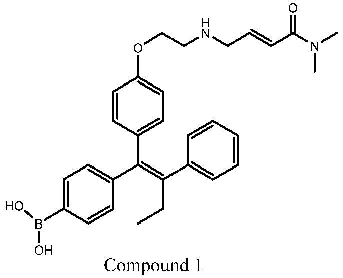

The synthesis scheme used for making Compound 1 is illustrated in FIG. 1.

### Example 2

As an illustrative example of a compound of formula (II), the following compound, denoted **Compound 2,** was synthesized:

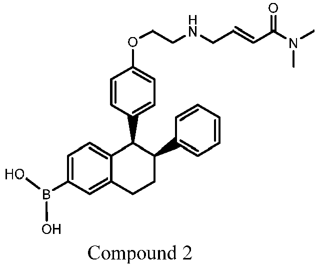

The synthesis scheme used for making Compound 2 is illustrated in FIG. 2.

### Comparative Example 3

As an illustrative example of a compound of formula (III), the following compound, denoted **Compound 3,** was synthesized:

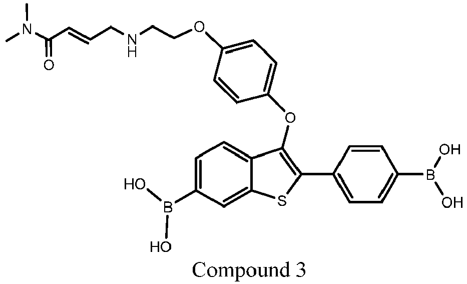

The synthesis scheme used for making Compound 3 is illustrated in FIG. 3.

### Comparative Example 4

As another illustrative example of a compound of formula (III), the following compound, denoted **Compound 4,** was synthesized:

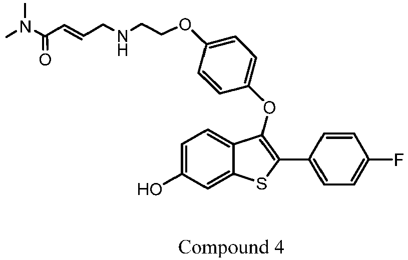

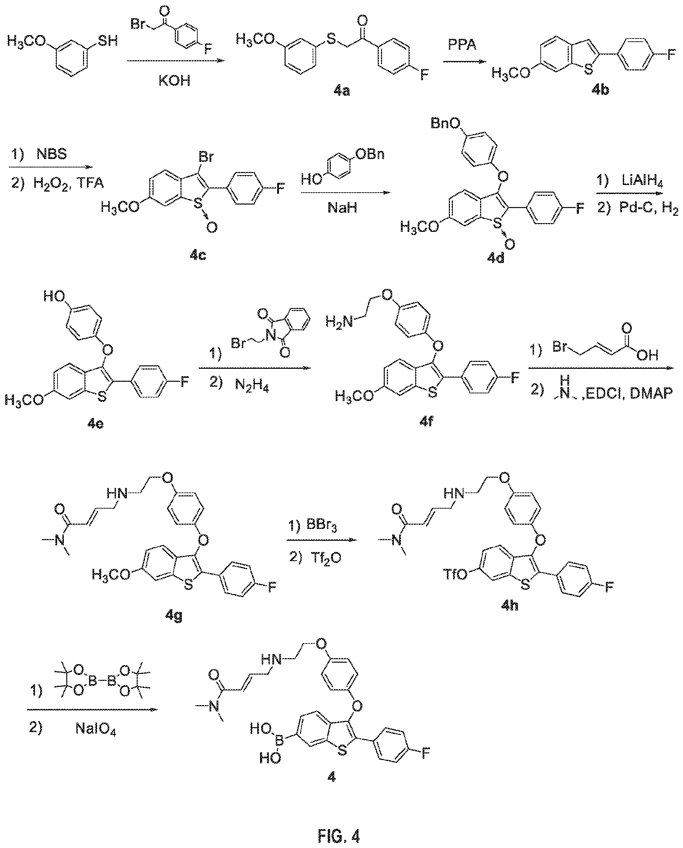

The synthesis scheme used for making Compound 4 is illustrated in FIG. 4.

### Comparative Example 5

As another illustrative example of a compound of formula (III), the following compound, denoted **Compound 5,** was synthesized:

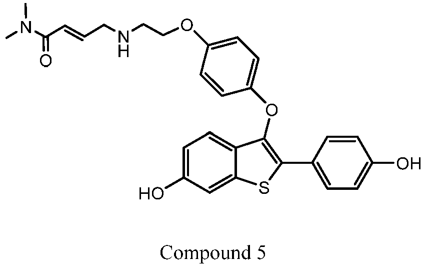

### Comparative Example 6

As another illustrative example of a compound of formula (III), the following compound, denoted **Compound 6,** was synthesized:

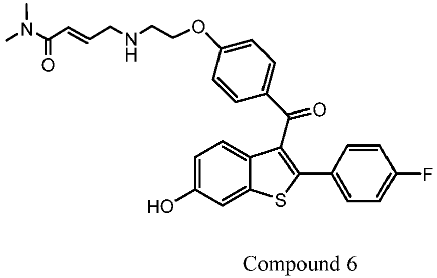

### Comparative Example 7

As another illustrative example of a compound of formula (III), the following compound, denoted **Compound 7,** was synthesized:

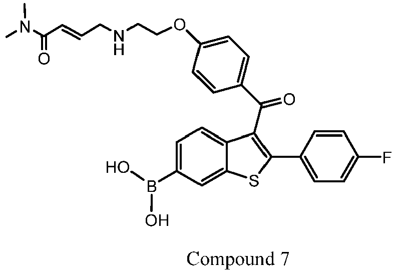

### Comparative Example 8

As another illustrative example of a compound of formula (III), the following compound, denoted **Compound 8,** was synthesized:

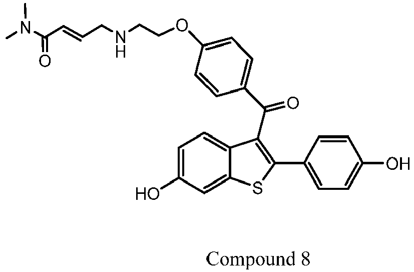

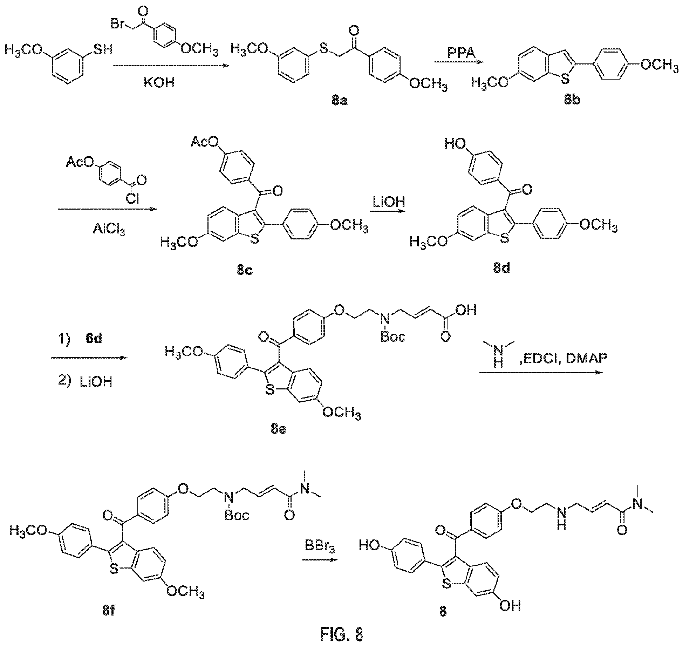

The synthesis scheme used for making Compound 8 is illustrated in FIG. 8.

### Example 9

As an illustrative example of a compound of formula (IV), the following compound, denoted **Compound 9,** was synthesized:

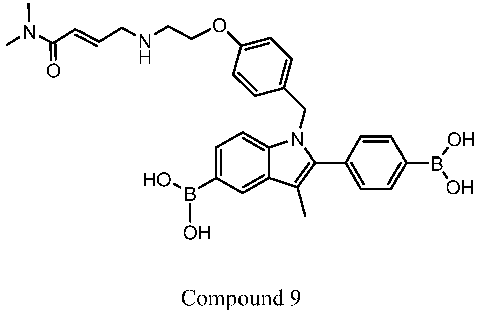

The synthesis scheme used for making Compound 9 is illustrated in FIG. 9.

### Comparative Example 10

As another illustrative example of a compound of formula (IV), the following compound, denoted **Compound 10,** was synthesized:

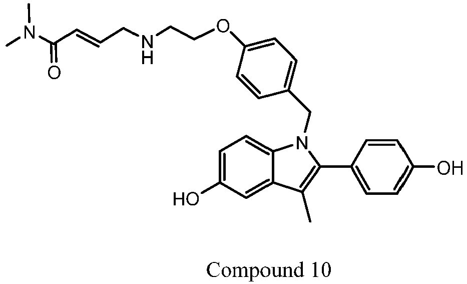

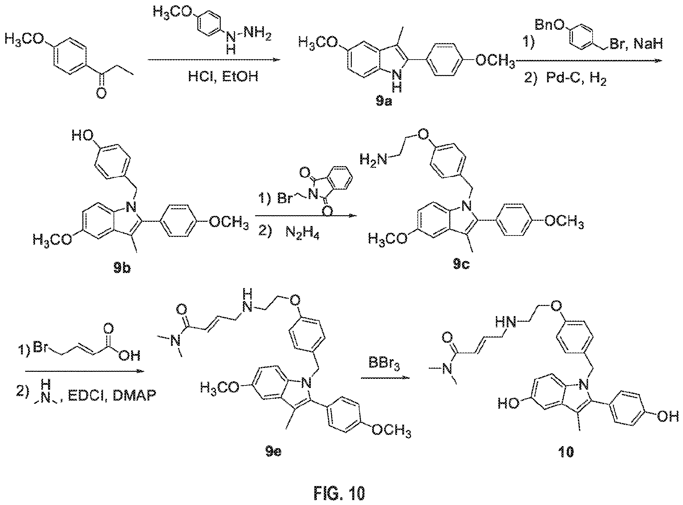

The synthesis scheme used for making Compound 10 is illustrated in FIG. 10.

### Comparative Example 11

As another illustrative example of a compound of formula (IV), the following compound, denoted **Compound 11,** was synthesized:

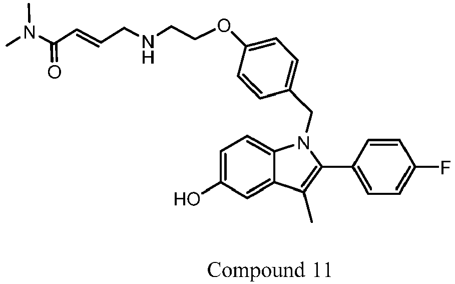

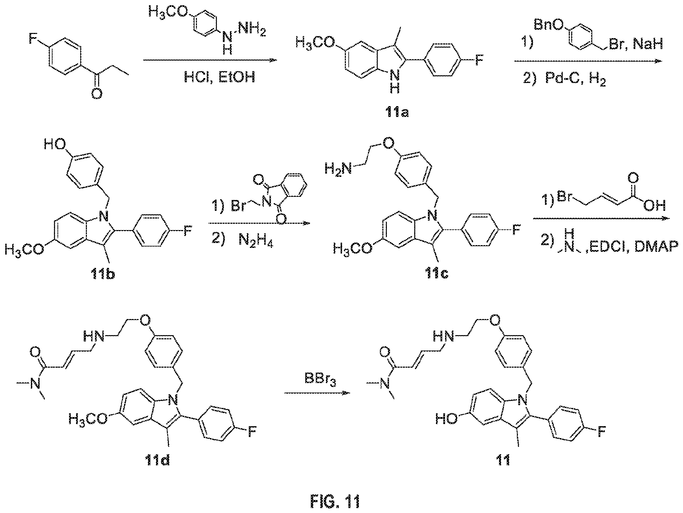

The synthesis scheme used for making Compound 11 is illustrated in FIG. 11.

### Comparative Example 12

As another illustrative example of a compound of formula (IV), the following compound, denoted **Compound 12,** was synthesized:

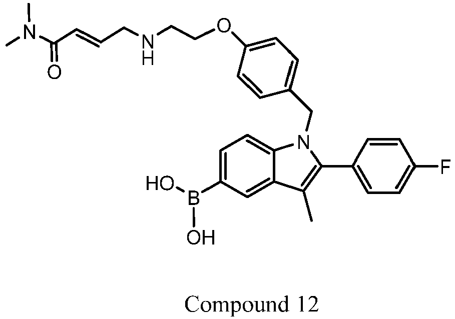

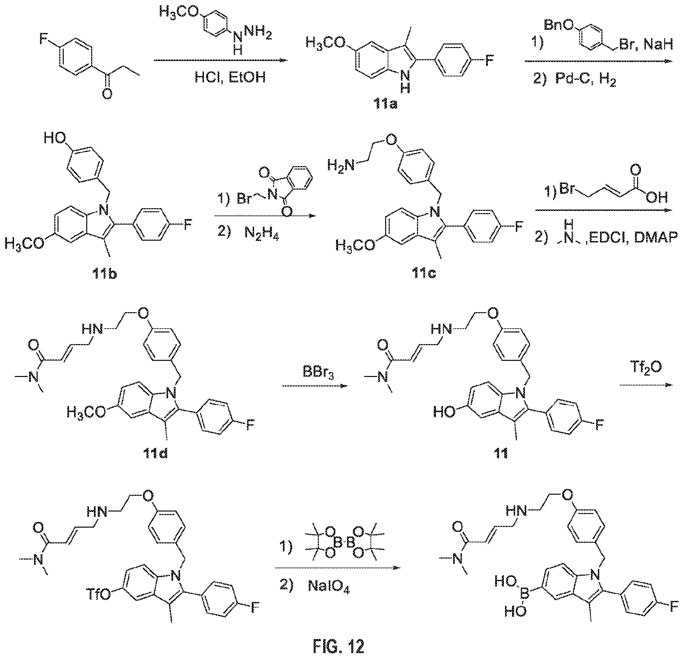

The synthesis scheme used for making Compound 12 is illustrated in FIG. 12.

### Comparative Example 13

As an illustrative example of a compound of formula (V), the following compound, denoted **Compound 13,** was synthesized:

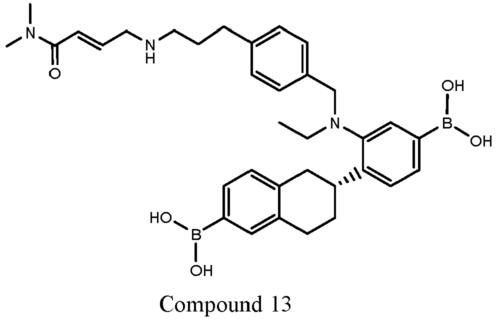

The synthesis scheme used for making Compound 13 is illustrated in FIG. 13.

### Comparative Example 14

As an illustrative example of a compound of formula (VI), the following compound, denoted **Compound 14,** was synthesized:

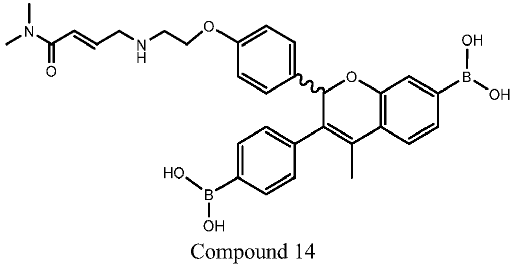

The synthesis scheme used for making Compound 14 is illustrated in FIG. 14.

### Comparative Example 15

As a further illustrative example of compounds of formula (III), the following compounds, denoted **Compound 15 and Compound 16,** were synthesized:

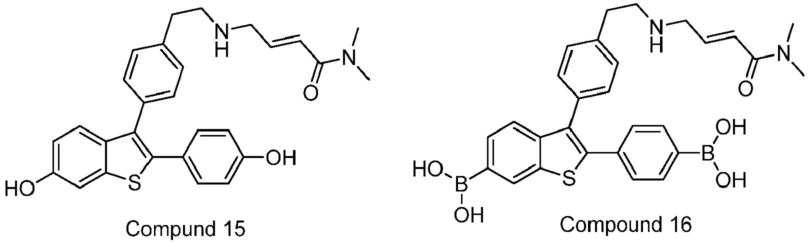

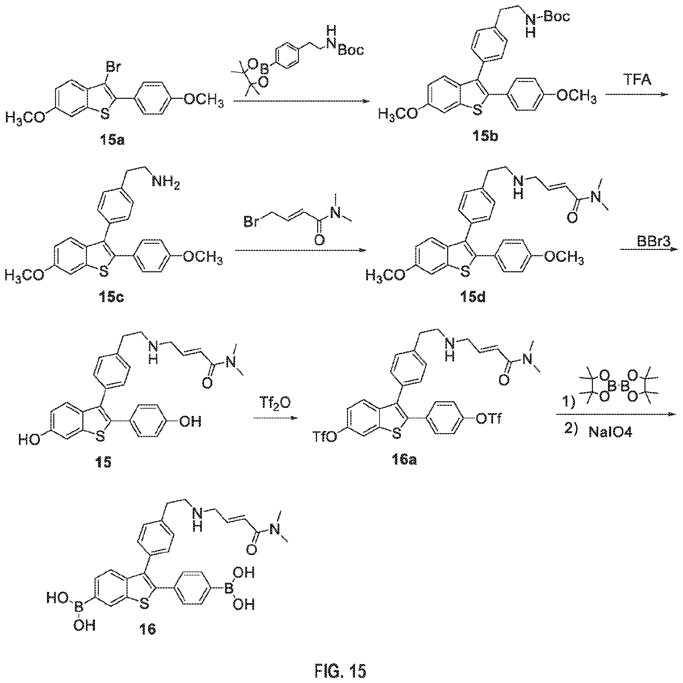

The synthesis scheme used for making Compound 15 and Compound 16 are illustrated in FIG. 15.

### Example 16

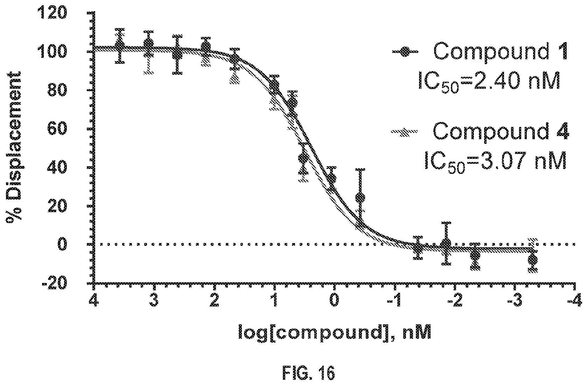

To evaluate the binding affinity of the disclosed ER targeting antagonists to the estrogen receptor, the LanthaScreen TR-FRET assay (Life Technologies) was used in which the test compounds compete with a fluomone ligand and the percent displacement was quantitatively correlated to the fluorescence intensity from the displaced tracer. FIG. 16 shows the competitive binding curves of compound **1** (an example of formula I) and compound **4** (an example of formula III) with IC50 values measured at 2.40 nM and 3.07 nM, respectively.

### Example 17

The ER antagonism of compound **1** is irreversible

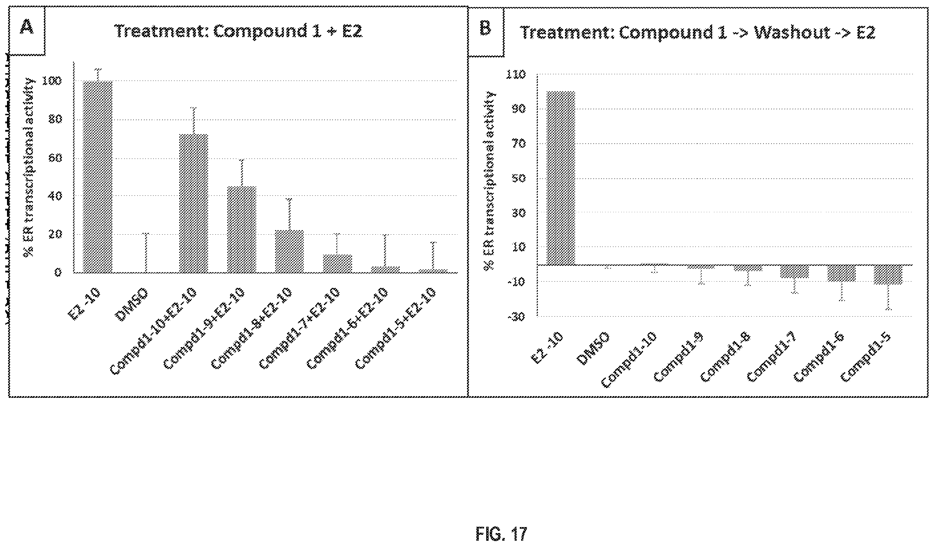

To confirm the irreversible nature of compound antagonism in ER+ breast cancer, we designed a washout experiment in which the ER mediated transcriptional activities were measured by the T47D-kb-Luc stably transfected human breast cancer cell reporter gene assay [7] treated with compound **1** either simultaneously with E2 or with E2 after a washout period. When compound **1** of varying doses and E2 (0.1 nM) were added to T47D-ERE-Luc cells at the same time, inhibition of ERE luciferase signal showed a dose-dependent manner as expected (FIG. 17, panel A). However, when cells were first treated with compound **1** for 1 hour, followed by a washout period of 3 hours in which cells were washed with fresh media every 30 min, and then treated with 0.1 nM E2, cells remained inhibited with minimal stimulation effect by E2 (FIG. 17, panel B). In other words, pre-treatment of compound **1** allowed ER to be irreversibly occupied and subsequent rescue by E2 is no longer possible. These data clearly show that compound **1** activity as an antagonist is irreversible as the molecule is permanently bound to ER.

### Example 18

### Compound 1 inhibits proliferation of breast cancer cells

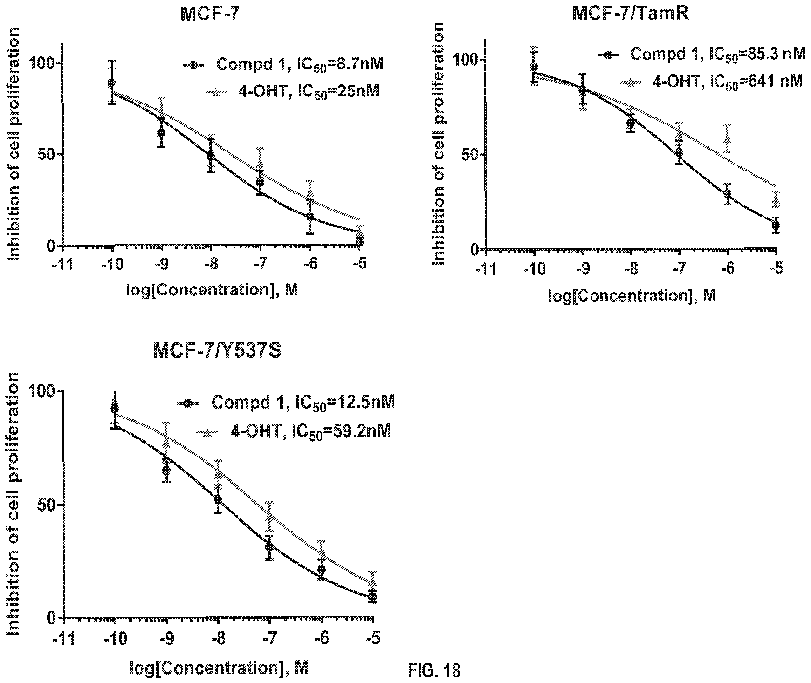

To assess the effect of compound **1** on breast cancer cell proliferation, MCF-7, MCF-7/TamR (*tamoxifen resistant*), and MCF-7(Y537S) (*expressing mutant ER*) were treated with compound **1** and 4-hydroxytamoxifen (4OHT) to determine the antiproliferative activity of an irreversible and reversible SERM. As shown in FIG. 18.

### Example 19

### Compound 1 inhibits xenograft breast tumor growth in mice

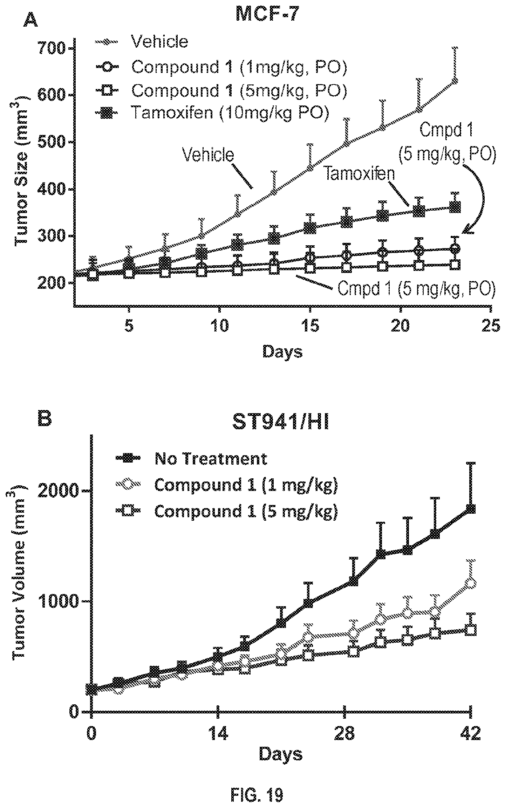

To determine the in vivo efficacy of compound **1** in inhibiting tumor growth, we treated mice bearing MCF-7 derived xenograft breast tumors with compound **1.** At 1 and 5 mg/kg daily oral doses, the MCF-7 tumor growth was nearly completely blocked during the three weeks of treatment time (FIG. 19, panel A). In comparison, treatment of the reversible SERM tamoxifen resulted in an inhibition of tumor growth that was significantly less efficacious than the compound **1** treatment groups, indicating that the targeted covalent bonding formation between compound **1** and C530 within the ER LBD confers enhanced anti-tumor efficacy over a conventional antiestrogen. In a patient derived xenograft metastasis tumor model (ST941/HI, ESR1Y537S), treatment with compound **1** at 1 mg/kg and 5 mg/kg doses resulted in significant growth inhibition (FIG. 19, panel B), demonstrating the therapeutic efficacy of the irreversible ER inhibitor in metastasized breast tumor resistant to endocrine treatment.

### Example 20

### Compound 1, but not compound 4, shows agonist activity in mouse uterus

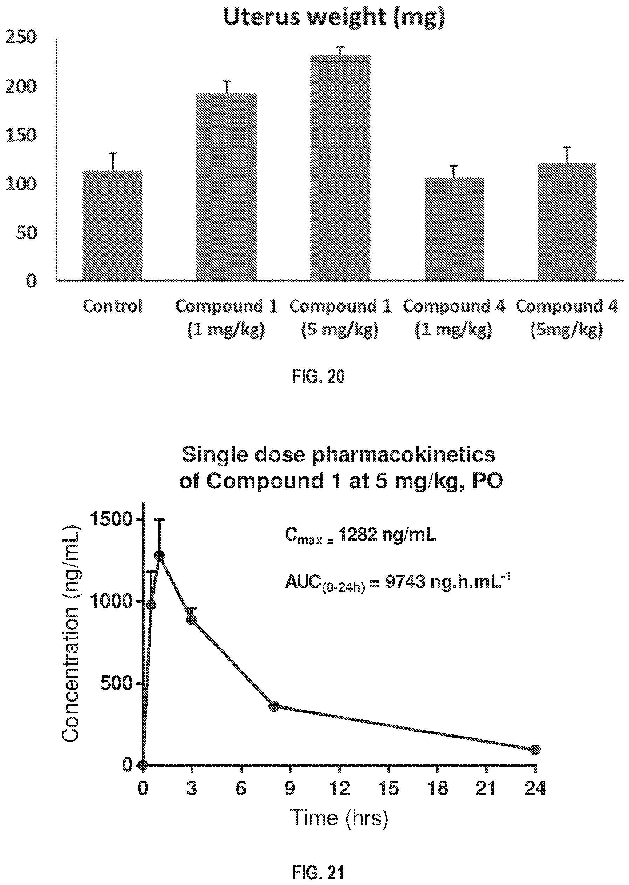

To assess the uterotrophic effect of the synthesized irreversible inhibitors, compound 1 and compound **4**, we treated 5-6 week old female NU/NU mice with **1** or **4** for 5 days and measured the uterine weights post treatment. It was found, as shown in FIG. 20, that compound **1** significantly increased uterine wet weight while compound **4** did not. This differing antagonist/agonist selectivity of **1** and 4 based on two different ER binding core structural motif demonstrates that it is possible to achieve the desired selectivity profile in irreversible inhibitors.

### Example 21

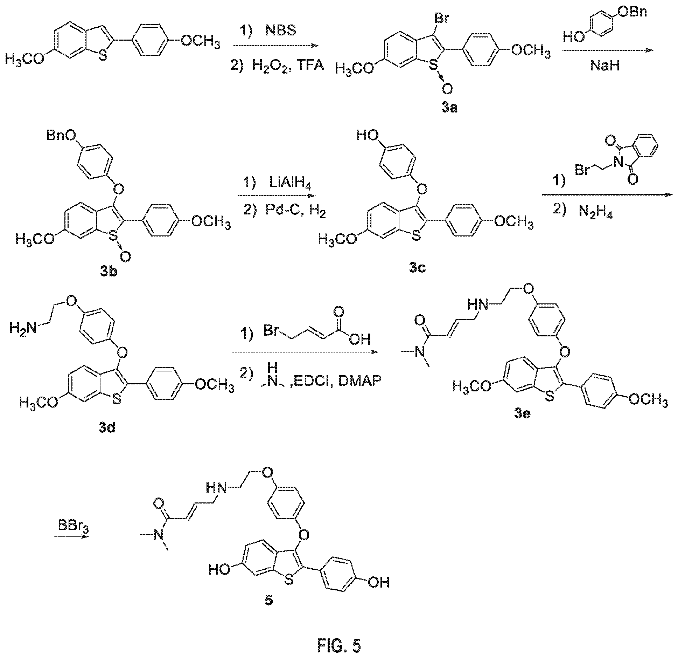

To test the oral bioavailability and metabolic stability of compound **1,** a single oral dose of 5 mg/kg was administered to Sprague Dawley rats and plasma samples were collected at various time intervals to measure compound **1** concentrations. The PK profile of compound **1** is plotted in FIG. 5. Peak concentration exceeded 1200 ng/mL with AUC value approaching 10,000 ng.h.mL-1, indicating excellent drug exposure profile. The strong showing of ZB499 oral bioavailability is attributable to the boronic acid moiety that has the dual capability of minimizing first-past metabolism and enhanced absorption.

### Example 22

We tested the antagonistic activities of compound 1 and compound 4 in an estrogen receptor positive (ER+) breast cancer cell line, MCF7 that is sensitive to antiestrogen treatment and its derivative cell lines that have become resistant to antiestrogen treatment. The cells were treated with a known SERM, 4-hydroxytamoxifen, a SERD, fulvestrant, compound 1, and compound 4, respectively at various doses to obtain the IC50 values of each treatment, defined as the concentration of the compound required to inhibit cell proliferation by 50%. Results are shown in Table 1.

**Table 1. Antiproliferative efficacy of example compounds in various breast cancer cell lines**

| | MCF7 | MCF7-TamR | MCF7-OHT | MCF7-F | MCF7-Y537S |
|---|---|---|---|---|---|
| | IC₅₀ (nM) | | | | |
| 4-Hydroxytamoxifen | 4.32 | 1090 | 3166 | 2572 | 1726 |
| Fulvestrant | 0.91 | 9.84 | 2.36 | 533 | 17.09 |
| Compound **1** | 3.03 | 11.76 | 1.97 | 29.88 | 8.31 |
| Compound **4** | 1.78 | 6.53 | 3.17 | 10.91 | 5.25 |

### References Cited:

1. Jasani B, Douglas-Jones A, Rhodes A, Wozniak S, Barrett-Lee PJ, Gee J, Nicholson R. Measurement of estrogen receptor status by immunocytochemistry in paraffin wax sections. Methods Mol Med. 2006;120:127-46.
2. Setiawan VW, Monroe KR, Wilkens LR, Kolonel LN, Pike MC, Henderson BE. Breast cancer risk factors defined by estrogen and progesterone receptor status: the multiethnic cohort study. Am J Epidemiol. 2009 May 15;169(10):1251-9.
3. Barrios C, Forbes JF, Jonat W, Conte P, Gradishar W, Buzdar A, Gelmon K, Gnant M, Bonneterre J, Toi M, Hudis C, Robertson JF. The sequential use of endocrine treatment for advanced breast cancer: where are we? Ann Oncol. 2012, 23(6):1378-86.
4. Nabholtz JM, Buzdar A, Pollak M et al. Anastrozole is superior to tamoxifen as first-line therapy for advanced breast cancer in postmenopausal women: results of a North American multicenter randomized trial. Arimidex Study Group. J Clin Oncol 2000; 18: 3758-3767.
5. Nabholtz JM, Bonneterre J, Buzdar A et al. Anastrozole (Arimidex) versus tamoxifen as first-line therapy for advanced breast cancer in postmenopausal women: survival analysis and updated safety results. Eur J Cancer 2003; 39: 1684-1689.
6. Hammond et al., Immunohistochemical Testing of Estrogen and Progesterone Receptors in Breast Cancer, Journal of Clinical Oncology, Vol 28, Issue 16, pp 2784-2795 (2010).
7. Wilson VS, Bobseine K, Gray LE Jr. Development and characterization of a cell line that stably expresses an estrogen-responsive luciferase reporter for the detection of estrogen receptor agonist and antagonists. Toxicol Sci. 2004, Sep; 81(1):69-77.

## Claims

1. A compound of Formula (I): 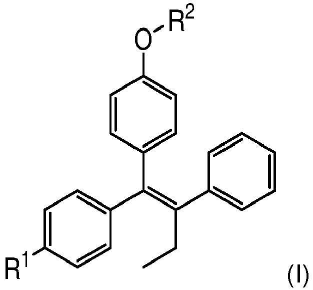 where:
R1 = 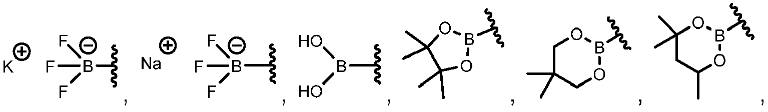 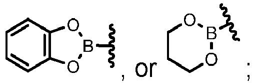
R2 = 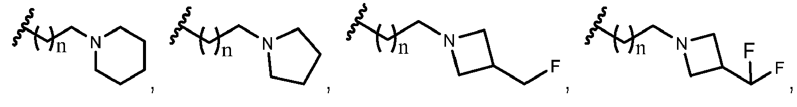 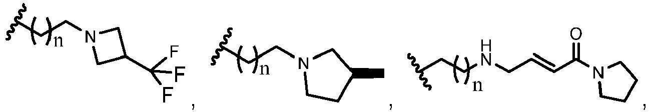 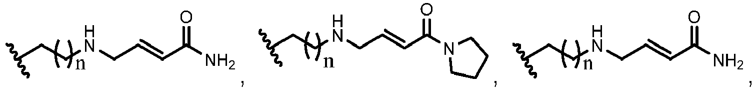 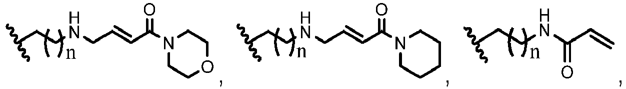 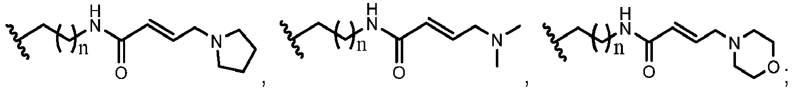
n = 1-3
wherein the R¹ substituent point of attachment is on the substituent boron atom of R¹, or a salt thereof, a solvate thereof, or a solvate of a salt thereof.

2. A pharmaceutical composition comprising a compound, pharmaceutically acceptable salt, solvate, or composition of claim 1 and a pharmaceutically acceptable carrier.

3. The pharmaceutical composition of claim 2, suitable for enteral administration, preferably oral administration.

4. The pharmaceutical composition of claim 2, suitable for parenteral administration.

5. A compound, pharmaceutically acceptable salt, solvate, or composition of claim 1 or a pharmaceutical composition of any of claims 2 to 4 for use in the treatment of a proliferative disease in a subject.

6. The compound for use according to claim 5 wherein the proliferative disease is cancer.

7. The compound for use according to claim 6 wherein the cancer is breast cancer.

8. The compound for use according to claim 7, wherein said breast cancer is an ER-positive breast cancer.

9. The compound for use according to claim 8, where said subject expresses a mutant ER-α protein.

## Patentansprüche

1. Eine Verbindung der Formel (I): 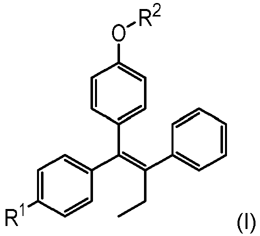 wobei:
R1 = 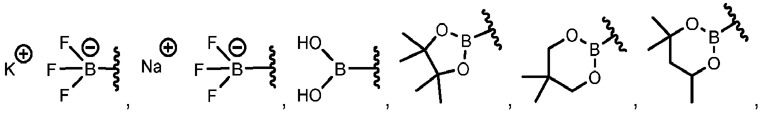 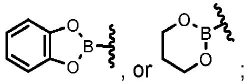
R2 = 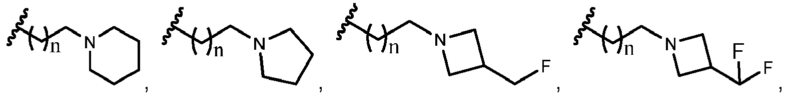 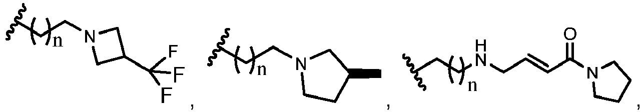 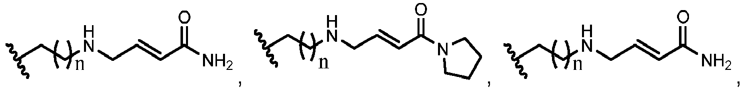 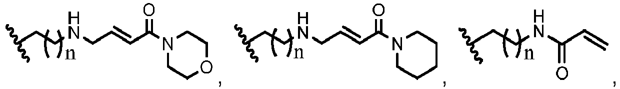 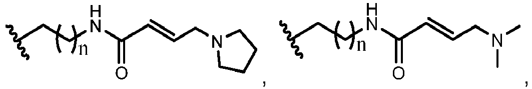 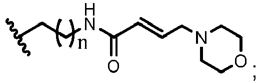
n = 1-3,
wobei der R¹-Substituenten-Bindungspunkt an dem Substituenten-Boratom von R¹ liegt, oder ein Salz davon, ein Solvat davon oder ein Solvat eines Salzes davon.

2. Eine pharmazeutische Zusammensetzung, die eine Verbindung, ein pharmazeutisch akzeptables Salz, Solvat oder eine pharmazeutisch akzeptable Zusammensetzung gemäß Anspruch 1 und einen pharmazeutisch akzeptablen Träger beinhaltet.

3. Pharmazeutische Zusammensetzung gemäß Anspruch 2, die zur enteralen Verabreichung, vorzugsweise zur oralen Verabreichung, geeignet ist.

4. Pharmazeutische Zusammensetzung gemäß Anspruch 2, die zur parenteralen Verabreichung geeignet ist.

5. Eine Verbindung, ein pharmazeutisch akzeptables Salz, Solvat oder eine pharmazeutisch akzeptable Zusammensetzung gemäß Anspruch 1 oder eine pharmazeutische Zusammensetzung gemäß einem der Ansprüche 2 bis 4 zur Verwendung bei der Behandlung einer proliferativen Erkrankung bei einem Individuum.

6. Verbindung zur Verwendung gemäß Anspruch 5, wobei die proliferative Erkrankung Krebs ist.

7. Verbindung zur Verwendung gemäß Anspruch 6, wobei der Krebs Brustkrebs ist.

8. Verbindung zur Verwendung gemäß Anspruch 7, wobei der Brustkrebs ein ER-positiver Brustkrebs ist.

9. Verbindung zur Verwendung gemäß Anspruch 8, wobei das Individuum ein mutiertes ER-α-Protein exprimiert.

## Revendications

1. Un composé de Formule (I) : 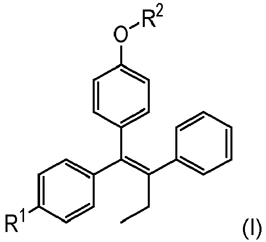 où :
R1 = 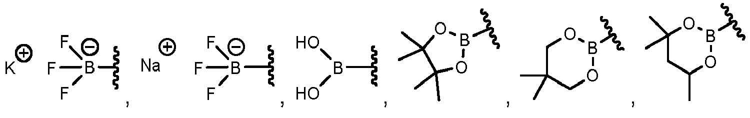 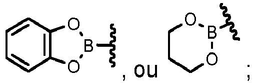
R2 = 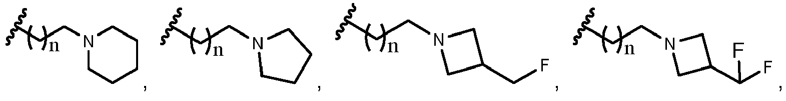 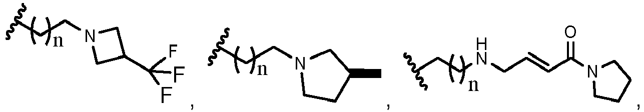 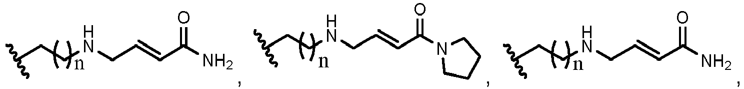 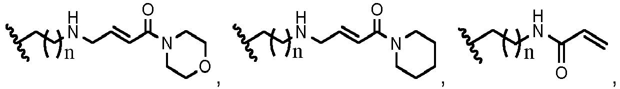 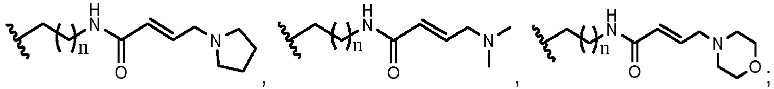
n = 1 à 3
dans lequel le point d'attache du substituant R¹ est sur l'atome de bore substituant de R¹, ou un sel de celui-ci, un solvate de celui-ci, ou un solvate d'un sel de celui-ci.

2. Une composition pharmaceutique comprenant un composé, un sel, solvate, ou une composition pharmaceutiquement acceptables de la revendication 1 et un support pharmaceutiquement acceptable.

3. La composition pharmaceutique de la revendication 2, convenant pour une administration entérale, de préférence une administration orale.

4. La composition pharmaceutique de la revendication 2, convenant pour une administration parentérale.

5. Un composé, un sel, solvate, ou une composition pharmaceutiquement acceptables de la revendication 1 ou une composition pharmaceutique de n'importe lesquelles des revendications 2 à 4 pour une utilisation dans le traitement d'une maladie proliférative chez un sujet.

6. Le composé pour une utilisation selon la revendication 5 dans lequel la maladie proliférative est un cancer.

7. Le composé pour une utilisation selon la revendication 6 dans lequel le cancer est un cancer du sein.

8. Le composé pour une utilisation selon la revendication 7, dans lequel ledit cancer du sein est un cancer du sein ER-positif.

9. Le composé pour une utilisation selon la revendication 8, où ledit sujet exprime une protéine ER-α mutante.
